Europäisches Patentamt

European Patent Office

Office européen des brevets

(19)

(11) Veröffentlichungsnummer: **0 218 809**
**B1**

(12) **EUROPÄISCHE PATENTSCHRIFT**

(45) Veröffentlichungstag der Patentschrift:
**11.10.89**

(51) Int. Cl.⁴: **A61B 17/36, A61B 17/22**

(21) Anmeldenummer: **86109737.6**

(22) Anmeldetag: **16.07.86**

(54) Führungssonde.

(30) Priorität: **19.09.85 DE 3533452**

(43) Veröffentlichungstag der Anmeldung:
**22.04.87 Patentblatt 87/17**

(45) Bekanntmachung des Hinweises auf die Patenterteilung:
**11.10.89 Patentblatt 89/41**

(84) Benannte Vertragsstaaten:
**AT BE CH DE FR GB IT LI LU NL SE**

(56) Entgegenhaltungen:
**WO-A-84/01294**
**DE-U- 8 517 084**

(73) Patentinhaber: **Messerschmitt-Bölkow-Blohm
Gesellschaft mit beschränkter Haftung,
Robert-Koch-Strasse, D-8012 Ottobrunn(DE)**

(72) Erfinder: **Hochberger, Jürgen,
Gerhart-Hauptmann-Strasse 15/XI,
D-8520 Erlangen(DE)**
Erfinder: **Ell, Christian, Dr. med., Burgbergstrasse,
D-8520 Erlangen(DE)**

ACTORUM AG

## Beschreibung

In der Medizin wird Laserlicht u.a. dazu verwendet, Gewebe gezielt zu koagulieren bzw. zu verdampfen und damit abzutragen. So liegt ein besonderer Vorteil von Laserlicht bestimmter Wellenlänge in der Übertragbarkeit über flexible Lichtleiter, was ein Arbeiten mit Laserlicht unter optischer Kontrolle mittels eines Endoskopes auch an sonst schwer zugänglichen Stellen im menschlichen Körper, gestattet. So wird Laserendoskopie in der Gastroenterologie, Urologie, Neurochirurgie und Pulmonologie etc. bereits in vielen Kliniken routinemäßig zum Abtragen von gut- und bösartigem Gewebe verwandt.

Wird nun beispielsweise im Bereich des Magen-Darmtraktes ein "röhrenförmiger" Gang durch das Wachstum eines Tumors oder andere Ursachen eingeengt, so kann er oft mittels Laserlicht unter endoskopischer Kontrolle wiedereröffnet werden. Besondere Schwierigkeiten bereiten dabei hochgradige und langstreckige Einengungen (Stenosen).

Für den oberen Verdauungstrakt sind dabei verschiedene Vorgehensweisen bekannt:

Ist eine Engstelle endoskopisch nicht durchgängig, so wird sie in Vorwärtsrichtung mittels Laserlicht eröffnet. Der natürliche Verlauf des Ganges ist dabei vor allem bei langstreckigen, bösartigen Tumorstenosen durch unregelmäßiges Wachstum der Geschwulst, überschießende Gewebsneubildung neben kraterartigem Tumorzerfall, oft nur noch sehr schwer zu erkennen, so daß ein Abweichen vom natürlichen Gangverlauf und ein Eröffnen der Wand durch den Laserstrahl häufig gegeben ist. Das Perforationsrisiko beträgt nach Angaben von Fachleuten bis zu 30%.

Eine zweite Methode für den oberen Verdauungstrakt besteht in einer kombinierten Laser-Bougierungstherapie, wobei im Falle der endoskopischen Unpassierbarkeit einer Engstelle zunächst diese durch Bougierung aufgedehnt wird, das Endoskop durch die Engstelle geführt wird und dann die Stenose unter langsamem Zurückziehen des Endoskops von distal nach proximal "aufgelasert" wird.

In vielen endoskopisch erreichbaren Stellen, wie beispielsweise im unteren Verdauungstrakt, im Bronchialtrakt oder in der Neurochirurgie, ist jedoch eine Bougierungsbehandlung kaum möglich – für den Patienten ist sie meist unangenehm und schmerzhaft – sowie mit einem gewissen Perforationsrisiko behaftet und macht meist mehrere Sitzungen an verschiedenen Tagen nötig, was die Verweildauer im Krankenhaus zusätzlich erhöht.

Die Nachteile der bekannten Einrichtungen soll die Erfindung vermeiden, insbesondere soll eine Führungssonde geschaffen werden, mit deren Hilfe eine Lokalisierung des Behandlungsortes sowie die Behandlungsrichtung vorgegeben und das Endoskop in dem Kanal auch während der Behandlung mit Laserlicht geführt werden kann. Diese Aufgabe wird durch eine nach den kennzeichnenden Merkmalen des Patentanspruchs 1 ausgebildete Führungssonde gelöst. Vorteilhafte Ausgestaltungen sind in den Unteransprüchen aufgeführt.

Die erfindungsgemäße, laserfeste Führungssonde wird zunächst, gewöhnlich unter endoskopischer Kontrolle oder radiologischer Darstellung, in die Engstelle eingeführt. Orientiert sich der Endoskopiker nun beim Vorgehen mit Endoskop und Laserstrahl an der laserfesten Sonde, so ist er sicher, dem natürlichen Gangverlauf zu folgen, was in einer Verringerung des Perforationsrisikos resultiert.

Eine derartige, laserfeste Sonde kann nun unabhängig von der jeweiligen Lokalisation an prinzipiell jeder mit dem Laserstrahl erreichbaren Stelle eingesetzt werden, um bei der Eröffnung einer Engstelle mittels Laserstrahl eine sichere Orientierungshilfe zu bieten.

Die Erfindung wird im folgenden anhand der in den Figuren teilweise schematisch dargestellten Ausführungsbeispiel näher beschrieben.

Es zeigen:

Fig. 1 einen Längsschnitt durch eine Führungssonde;

Fig. 2 einen Längsschnitt durch den laserfesten Teil einer Führungssonde;

Fig. 3 einen Längsschnitt durch den laserfesten Teil einer Führungssonde mit aneinandergehängten Elementen;

Fig. 4 (a–e) die Anwendung einer Führungssonde zur laserendoskopischen Behandlung einer Stenose.

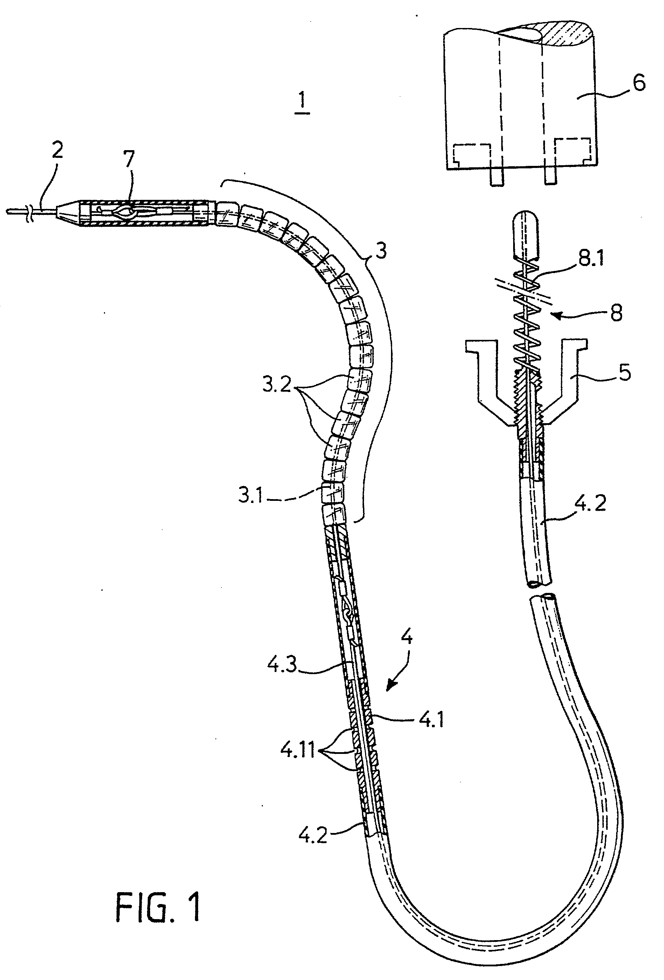

Die in Fig. 1 dargestellte Führungssonde 1 besteht im wesentlichen aus einer flexiblen Führungsspitze 2, einem flexiblen, laserfesten Teilstück 3 sowie einem Verlängerungsteil 4 mit einer Kupplung 5 zum Anschluß an ein entsprechendes Kupplungsstück 6 einer Absaugeinrichtung. Die Führungsspitze 2 besteht aus einem vergleichsweise dünnen, flexiblen, korrosionsfesten Draht, der wahlweise über eine Verbindung 7 an das laserfeste Teilstück 3 angekoppelt werden kann und das Einführen der Sonde in eine Engstelle dadurch erleichtern soll, indem er dem im allgemeinen weniger flexibleren Teilstück 3 als Pfadfinder dient. Die Länge dieser Spitze 2 beträgt vorteilhafterweise ca. 10 cm.

Das sich an die Führungsspitze 2 anschließende, laserfeste Teilstück 3 besteht aus einem Kerndraht 3.1, auf den zylinderförmige Elemente 3.2 nach Art einer Perlenkette aufgereiht sind. Die Elemente 3.2 bestehen aus einem hochtemperaturfesten Werkstoff, wie z.B. Quarzglas oder Aluminiumoxydkeramik, dessen Schmelzpunkt möglichst über 1000° C liegt und außerdem das jeweils verwendete Laserlicht in einem möglichst geringen Maße absorbiert. Chemisch sollte sich der Werkstoff möglichst inert gegenüber dem umliegenden Gewebe und den bei der Verbrennung durch das Laserlicht entstehenden Stoffen verhalten.

Der Kerndraht 3.1 selbst sollte ähnliche Eigenschaften wie die Elemente 3.2 haben, also ein möglichst geringes Absorptions- bzw. hohes Reflexionsvermögen gegenüber dem verwendeten Laserlicht einen hohen Schmelzpunkt sowie ein chemisch möglichst inertes Verhalten. Als vorteilhafte Ausführung hierfür hat sich ein Wolframdraht mit einem Durchmesser von 0,5 mm erwiesen, auf den eine Reinstgoldschicht von ca. 30 μ unter Verwendung von Hartgold als Haftvermittler galvanisch aufgetragen wird.

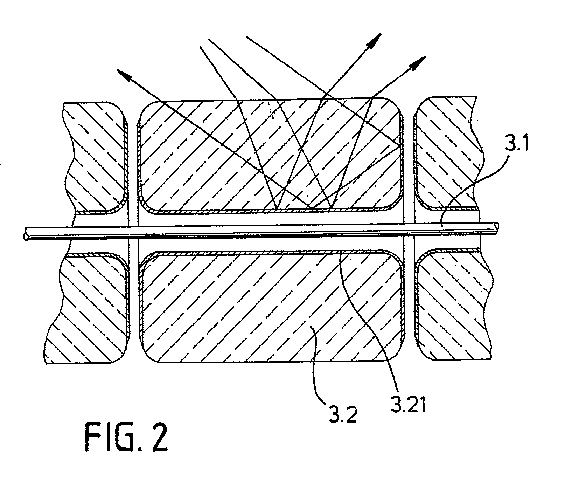

Anstelle von Wolfram ist auch ein entsprechend vergoldeter Draht aus einer Platin-Rhodiumlegierung (70/30) geeignet. Um den Kerndraht 3.1 zusätzlich vor dem Einfluß des Laserlichtes zu schützen, können, wie in Fig. 2 dargestellt, die zylinderförmigen Elemente 3.2 an den Stirnflächen sowie an den Wandungen der inneren Bohrung eine hochreflektierende Schicht 3.21, z.B. aus aufgedampften Gold mit einer Stärke von ca. 30 μ aufweisen. Das durch die weitestgehend transparenten Elemente 3.2 eindringende Laserlicht wird an diesen Schichten 3.21 wieder reflektiert und verläßt die Elemente, ohne auf den Kerndraht 3.1 zu gelangen.

Als vorteilhafte Dimensionierung für ein Element 3.2 für einen endoskopischen Einsatz der Führungssonde hat sich ein Außendurchmesser von ca. 2,5 mm, eine Innenbohrung von 0,7 mm sowie eine Länge von ca. 5 mm erwiesen. Das gesamte Teilstück 3 weist vorteilhafterweise ca. 30 derartige Elemente 3.2 auf.

Das sich an das Teilstück 3 anschließende Verlängerungsteil 4 besteht aus einem mit Schlitzen 4.11 versehenen Drainagerohr 4.1 mit gleichem Durchmesser wie das Teilstück 3 zur Absaugung der beim Abtragungsprozeß des Gewebes entstehenden Gase bzw. zur Spülung der Behandlungsstelle. Das Drainagerohr 4.1 mündet in einen flexiblen Kunststoffschlauch 4.2, z.B. aus Teflon, welcher am Ende ein Kupplungsstück 5 zum Anschluß an ein entsprechendes Kupplungsstück 6 einer nicht mehr dargestellten Absaug- bzw. Spüleinrichtung trägt. Das Verlängerungsteil 4 weist ebenfalls einen zentralen Draht, bzw. ein Drahtseil 4.3 auf, welches an einem Ende mit dem Kerndraht 3.1 verbunden ist. Das andere Ende des Drahtes 4.3 mündet in eine Spannvorrichtung 8, welche mittels einer Schraubenfeder 8.1 einen einstellbaren Zug ausübt. Durch diese Spannvorrichtung werden die Elemente 3.2 mittels des Drahtes 4.3 sowie des Kerndrahtes 3.1 zusammengeschoben und gegeneinander leicht verspannt. Dadurch erhält das Teilstück 3 eine besondere flexible Eigenschaft, die einerseits ein leichtes Abbiegen des Teilstückes 3 ermöglicht, andererseits eine leichte rückstellende Kraft auf dieses ausübt.

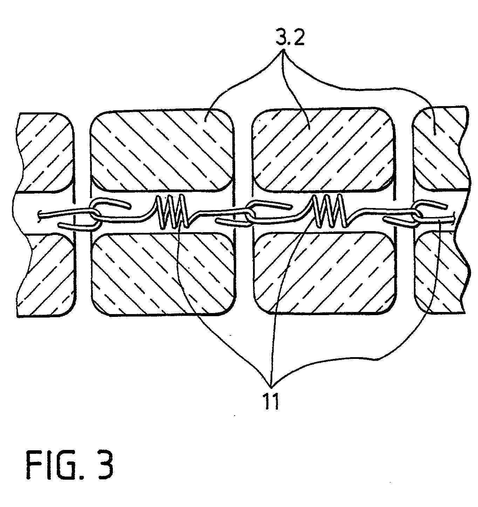

In Fig. 3 ist ein Ausführungsbeispiel eines laserfesten Teilstückes einer Führungssonde dargestellt, bei dem jedes zylindrische Element 3.2 ein zentrales, federndes Verbindungselement (11) aufweist, mittels welchem eine beliebige Anzahl von Elementen 3.2 aneinandergereiht bzw. ausgetauscht werden können.

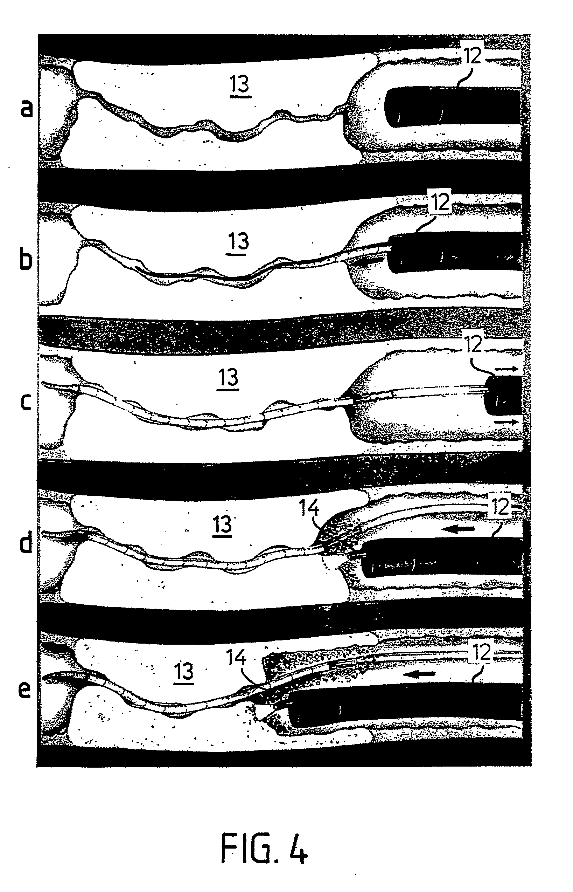

In Fig. 4 ist die Anwendung einer erfindungsgemäßen Führungssonde bei der laserendoskopischen Beseitigung einer Stenose beschrieben. Zuerst (Fig. 4a) wird ein gebräuchliches Endoskop 12 an den Beginn einer Stenose 13 aus wucherndem Gewebe geführt. Durch den Biopsiekanal des Endoskops 12 wird dann die erfindungsgemäße Führungssonde 1 unter Sicht bzw. unter radiologischer Kontrolle in die zu behandelnde Stenose eingeführt (Fig. 4b). Die Führungssonde wird dabei so weit vorgeschoben, daß das laserfeste Teilstück 3 in der Stenose 13 plaziert wird und das Drainagerohr 4.1 noch freiliegt (Fig. 4c). Da herkömmliche Endoskope lediglich einen Biopsiekanal aufweisen, wird das Endoskop 12 zurückgezogen, wobei die Führungssonde in der Stenose verbleibt (Fig. 4c). Der so freiwerdende Biopsiekanal wird mit einem Lichtleiter 14 bestückt, der mit einer Laserlichtquelle, z.B. einem Neodym-YAG-Laser, verbunden ist. Das so bestückte Endoskop 12 wird wieder an den Beginn der Stenose 13 geschoben (Fig. 4d), worauf dann der Operateur die Stenose um die Führungssonde herum abtragen kann, ohne daß dabei die Führungssonde selbst durch das Laserlicht beschädigt wird (Fig. 4e).

**Patentansprüche**

1. Führungssonde für die operative Endoskopie mit Laserlicht, dadurch gekennzeichnet, daß die Führungsonde (1) ein zumindest abschnittsweise flexibles aus hochtemperaturfesten Elementen bestehendes Teilstück (3) aufweist, in dessen direkt angrenzender Umgebung (13) die operative Behandlung mittels Laserlicht stattfindet und dessen Schmelz- bzw. Erweichungspunkt über derjenigen Temperatur liegt, welche durch Erwärmung aufgrund der Absorption direkter Bestrahlung des Teilstücks (3) mit dem Laserlicht bei der operativen Behandlung erreicht wird.

2. Führungssonde nach Anspruch 1, dadurch **gekennzeichnet**, daß das Teilstück (3) Elemente (3.2) aus Keramik, Glas oder Glaskeramik aufweist, welche nach Art einer Perlenkette auf einen Kerndraht (3.1) aufgereiht sind.

3. Führungssonde nach Anspruch 2, dadurch **gekennzeichnet**, daß der Kerndraht (3.1) aus einem Edelmetall oder einer -legierung, bzw. aus einer oder mehreren Glasfasern besteht.

4. Führungssonde nach Anspruch 2 oder 3, dadurch **gekennzeichnet**, daß der Kerndraht (3.1) und/oder die Elemente (3.2) zumindest teilweise mit einer das Laserlicht reflektierenden Schicht (3.21) versehen sind.

5. Führungssonde nach einem der Ansprüche 2 bis 4, dadurch **gekennzeichnet**, daß die Elemente (3.2) zylinderförmig ausgebildet sind und eine koaxiale Bohrung aufweisen.

6. Führungssonde nach Anspruch 1, dadurch **gekennzeichnet**, daß das Teilstück (3) Elemente (3.2) aus Keramik, Glas oder Glaskeramik aufweist, welche jeweils ein zentrales Verbindungselemente (11) zum Aneinanderreihen beinhalten.

7. Führungssonde nach einem der Ansprüche 1 bis 6, dadurch **gekennzeichnet**, daß das Teilstück (3) mit einer im Durchmesser dünneren, flexiblen Führungsspitze (2) versehen ist.

8. Führungssonde nach einem der Ansprüche 2 bis 7, dadurch **gekennzeichnet**, daß die Elemente (3.2) über eine Federeinrichtung (8) mittels des Kerndrahtes (3.1) bzw. der Verbindungselemente (11) in axialer Richtung zusammengehalten und gegeneinander verspannt werden.

9. Führungssonde nach einem der Ansprüche 2 bis 8, dadurch **gekennzeichnet,** daß die Elemente (3.2) nach Art von Heizdrahtperlen an ihren Stirn- und Endflächen sich gegenseitig überdeckende Kragenstücke aufweisen, derart, daß sie auch in gebogenem Zustand der Führungssonde den Kerndraht gegen das Laserlicht abschirmen.

10. Führungssonde nach einem der Ansprüche 1 bis 9, dadurch **gekennzeichnet,** daß das Teilstück (3) mit einem flexiblen Verlängerungsteil (4) zum direkten oder mittels eines Endoskopkanals, indirekten Einführen versehen ist.

11. Führungssonde nach Anspruch 10, dadurch **gekennzeichnet,** daß das Verlängerungsteil als rohr- und/oder schlauchförmige Leitung (4.1, 4.2) mit Öffnungen (4.11) zum Absaugen von Gasen und/oder Flüssigkeiten oder zur Spülung ausgebildet ist.

**Claims**

1. A guide probe for use in operative laser-endoscopy, characterised in that the guide probe (1) has an at least segmentally flexible part (3) made of high-temperature resistant elements, in the directly adjacent zone (13) of which the operative laser-beam treatment takes place and the melting or fusing temperature of which is higher than the temperature which is reached by heating up due to absorption of direct irradiation of said part (3) with the laser beam in the course of the operative treatment.

2. A guide probe according to Claim 1, characterised in that the part (3) has ceramic, glass or glass-ceramic elements (3.2), which are threaded on a core wire (3.1) in the manner of a string of beads.

3. A guide probe according to Claim 2, characterised in that the core wire (3.1) consists of a precious metal or precious metal alloy or of one or several glass fibres.

4. A guide probe according to Claim 2 or 3, characterised in that the core wire (3.1) and/or the elements (3.2) are provided at least partly with a coating (3.21) capable of reflecting laser light.

5. A guide probe according to any one of Claims 2 to 4, characterised in that the elements (3.2) are of cylindrical shape and have a coaxial bore.

6. A guide probe according to Claim 1, characterised in that the part (3) has elements (3.2) made of ceramic, glass or glass-ceramic, each of which comprises a central connecting member (11) for stringing.

7. A guide probe according to any one of Claims 1 to 6, characterised in that the part (3) is provided with a flexible lead point (2) having a narrower diameter.

8. A guide probe according to any one of Claims 2 to 7, characterised in that the elements (3.2) are held together in axial direction and are tensioned relative to each other through a spring device (8) by means of the core wire (3.1) or the connecting members (11), respectively.

9. A guide probe according to any one of Claims 2 to 8, characterised in that the elements (3.2), in the manner of heating-wire beads, have on their front and end surfaces mutually overlapping collar pieces in such a manner, that they shield the core wire from the laser beam even in the bent position of the guide probe.

10. A guide probe according to any one of Claims 1 to 9, characterised in that the part (3) is provided with a flexible extension (4) for either direct introduction or indirect introduction by means of an endoscope conduit.

11. A guide probe according to Claim 10, characterised in that the extension is constructed as a tubular and/or hose-like conduit (4.1, 4.2) with orifices for the evacuation by suction of gases and/or liquids or for rinsing.

**Revendications**

1. Sonde de guidage pour l'endoscopie opératoire à l'aide de lumière laser, caractérisée en ce que la sonde de guidage (1) présente un tronçon (3) flexible au moins par parties et composé d'éléments résistant à des températures élevées, dans l'entourage direct (13) duquel a lieu le traitement opératoire à l'aide de lumière laser et dont le point de fusion ou de ramollissement est supérieur à la température qui est atteinte lors du traitement opératoire avec la lumière laser du fait de l'échauffement dû à l'absorption du rayonnement direct par le tronçon (3).

2. Sonde de guidage selon la revendication 1, caractérisée en ce que le tronçon (3) présente des éléments (3.2) en céramique, en verre ou en vitrocéramique qui sont enfilés sur un fil central (3.1) à la manière d'un collier de perles.

3. Sonde de guidage selon la revendication 2, caractérisée en ce que le fil central (3.1) est constitué d'un métal précieux ou d'un alliage de métaux précieux, ou d'une ou plusieurs fibres de verre.

4. Sonde de guidage selon la revendication 2 ou 3, caractérisée en ce que le fil central (3.1) et/ou les éléments (3.2) sont munis au moins partiellement d'une couche (3.21) réfléchissant la lumière laser.

5. Sonde de guidage selon l'une des revendications 2 à 4, caractérisée en ce que les éléments (3.2) sont réalisés sous une forme cylindrique et présentent un alésage coaxial.

6. Sonde de guidage selon la revendication 1, caractérisée en ce que le tronçon (3) présente des éléments (3.2) en céramique, en verre ou en vitrocéramique qui contiennent chacun un élément central d'assemblage (11) permettant de les aligner les uns par rapport aux autres.

7. Sonde de guidage selon l'une des revendications 1 à 6, caractérisée en ce que le tronçon (3) est muni d'une pointe de guidage (2) flexible de plus faible diamètre.

8. Sonde de guidage selon l'une des revendications 2 à 7, caractérisée en ce que les éléments (3.2) sont maintenus réunis en direction axiale et tendus les uns par rapport aux autres par l'intermédiaire d'un dispositif à ressort (8) à l'aide du fil central (3.1) ou des éléments d'assemblage (11).

9. Sonde de guidage selon l'une des revendications 2 à 8, caractérisée en ce que les éléments (3.2) présentent à la manière de perles de filaments chauffants, sur leurs surfaces frontales et terminales, des embouts se recouvrant mutuellement de telle sorte qu'ils protègent le fil central de la lumière laser, même lorsque la sonde de guidage est pliée.

10. Sonde de guidage selon l'une des revendications 1 à 9, caractérisée en ce que le tronçon (3) est muni d'un prolongateur flexible (4) pour une introduction directe ou indirecte à l'aide d'un canal d'endoscope.

11. Sonde de guidage selon la revendication 10, caractérisée en ce que le prolongateur est réalisé sous la forme d'un conduit en forme de tube et/ou de tuyau (4.1, 4.2) muni d'ouvertures (4.11) pour l'aspiration de gaz et/ou des liquides ou pour le lavage.

FIG. 1

FIG. 2

3.2

11

FIG. 3

FIG. 4